# EUROPEAN PATENT APPLICATION

(11) **EP 4 026 888 A1**
(43) Date of publication of application: **13.07.2022**
(21) Application number: 20873204.0
(22) Date of filing: 30.09.2020
(51) Int. Cl.: C12M 1/00, C12M 3/00

(54) **CONTAINER FOR SEPARATING LIVE CELLS**

(30) Priority: 30.09.2019 JP 2019179002
(71) Applicant: TERUMO Kabushiki Kaisha, Tokyo 151-0072 (JP)
(72) Inventor: YUKI, Risa, Ashigarakami-gun, Kanagawa 259-0151 (JP); OYAMA, Kenji, Ashigarakami-gun, Kanagawa 259-0151 (JP); MATSUDA, Isamu, Ashigarakami-gun, Kanagawa 259-0151 (JP); OHASHI, Fumiya, Ashigarakami-gun, Kanagawa 259-0151 (JP)
(74) Representative: Prüfer & Partner mbB Patentanwälte · Rechtsanwälte
(86) International application number: PCT/JP2020/037090
(87) International publication number: WO 2021/065986

(57) **Abstract**

An object of the present invention is to provide a container for isolating living cells. The object is achieved by a container for crushing a sample by pressing, the container including a container main body having an opening part and a partition which separates an upper space and a lower space of the container main body, in which at least a part of the partition has a portion which is not horizontal with respect to the bottom of the container.

## Description

### Technical Field

The present invention relates to a method for isolating living cells from a tissue derived from a living body, a container to be used in the same method, a kit to be used in the same method, and a graft produced using living cells obtained by the same method.

### Background Art

Despite the recent innovative progress in the treatment of heart disease, a therapeutic system for severe heart failure has not been established yet. A medical therapy using a β blocker or an ACE inhibitor has been practiced as a treatment method for heart failure, however, in a case of heart failure which has become so severe that such treatment is not successful, a substitution therapy such as a ventricular assist device and heart transplantation, that is, a surgical treatment, has been practiced.

Meanwhile, as a solution for the treatment of severe heart failure, development of new regenerative medicine is considered to be indispensable in recent days. In a severe myocardial infarction the like, cardiomyocytes become dysfunctional, and proliferation of fibroblast cells and interstitial fibrosis proceed, thus causing heart failure. Cardiomyocytes are damaged and undergo apoptosis as heart failure progresses, however, since cardiomyocytes hardly undergo cell division, the number of the cardiomyocytes decreases, and the heart function is further reduced.

A cell transplantation method is useful for heart function recovery in a patient with such severe heart failure, and clinical application of the method using myoblast cells has already been initiated.

A three-dimensionally constructed cell culture which can be transplanted to a heart, the cell culture containing myoblast cells, and a method for producing the same have been recently provided as an example of the cell transplantation method (Patent Literature 1). The myoblast cells used in the cell transplantation are generally obtained by isolating CD56-positive cells such as myoblast cells and muscle satellite cells from a skeletal muscle tissue of a subject of the transplantation. As a strategy for increasing a proportion of the CD56-positive cells in the cells isolated from the skeletal muscle tissue, for example, a method of subjecting the skeletal muscle tissue to enzyme treatment by soaking the tissue in a proteolytic enzyme solution for a predetermined time and discarding the enzyme treatment solution thus obtained, and then subjecting the tissue to the enzyme treatment again by soaking the tissue in a proteolytic enzyme solution for a predetermined time and collecting cells contained in the enzyme treatment solution thus obtained is known (Patent Literature 2).

### Citation List

### Patent Literature

Patent Literature 1: JP 2007-528755 A
Patent Literature 2: JP 2011-110368 A

### Summary of Invention

### Technical Problem

Various isolation methods and isolation conditions have been devised for the isolation of living cells from a tissue derived from a living body. However, there are a variety of cells constituting various tissues from a living body. Among these, proportions of stem cells or progenitor cells are small in the tissues from a living body, and many of the stem cells or the progenitor cells are present in a particular niche. For example, a skeletal muscle tissue is formed of muscle fibers, and the substance of a muscle fiber is a multinucleated cell surrounded by a plasma membrane. However, since CD56-positive cells such as muscle satellite cells and/or myoblast cells, which are the progenitor cells of the multinucleated cells, are localized only between a basement membrane and a plasma membrane of the muscle fiber, it is necessary to crush the skeletal muscle tissue to the extent that the CD56-positive cells are not destructed, while loosening the binding of the basement membrane of the muscle fibers, in order to isolate the CD56-positive cells from the skeletal muscle tissue of a subject.

Up until now, a shredding (mincing) treatment and an enzymatic degradation treatment that are performed by hand have been mainly performed for the isolation of the CD56-positive cells from the skeletal muscle tissue. The operation of disrupting the muscle fibers by the shredding operation is complicated and requires long time, and variations between operators exist in the number of collected living cells or viability, since the determination depends on the intuition of the operators. A simple method for stably isolating various living cells from various tissues derived from a living body has not been found yet.

### Solution to Problem

As a result of diligent studies to solve the above-described problems, the present inventors found that the number of collected living cells, viability, and purity are increased in progenitor cells (hereinafter, also referred to as stem cells) and the like by crushing a tissue derived from a living body, particularly, a skeletal muscle tissue, by pressing, thereby completing the present invention.

The present invention relates to the following.
<1> A container for crushing a sample by pressing, the container including
   a container main body having an opening part and a partition which separates an upper space and a lower space of the container main body, in which at least a part of the partition has a portion which is not horizontal with respect to the bottom of the container.
<2> The container according to <1>, in which the lower space is configured so that a protruding part which protrudes towards the opening part is formed by the partition.
<3> The container according to <1> or <2>, in which the partition has a communicating hole at the apex thereof.
<4> The container according to any one of <1> to <3>, in which the partition has a shape that resembles an inverted V or a shape of two tilted lines converging at the top.
<5> The container according to <4>, in which slopes of the shape that resembles an inverted V and/or the shape of two tilted lines converging at the top are asymmetrical.
<6> The container according to any one of <1> to <5> further including a sealed part.
<7> The container according to any one of <1> to <6>, in which the partition is formed by seal formation.
<8> The container according to any one of <1> to <7>, in which a sample is a tissue derived from a living body.
<9> The container according to any one of <1> to <8>, which is for isolating CD56-positive cells.

### Advantageous Effects of Invention

Isolation time can be shortened, and the number of collected living cells, viability, and purity of stem cells can be increased while being stabilized by using the isolation method of the present invention and/or the container of the present invention for isolation.

### Brief Description of Drawings

Fig. 1 is a schematic diagram illustrating a muscle satellite cell (A) existing between a basement membrane (B) and a plasma membrane (C) of a muscle fiber which constitutes a skeletal muscle.
Fig. 2 illustrates an aspect of a container of the present invention for crushing a sample in which a protruding part that protrudes towards an opening part is formed on the left side of a lower space 7 by one partition.
Fig. 3 illustrates an aspect of the container of the present invention for crushing a sample in which the protruding part that protrudes towards the opening part is formed in the middle of the lower space 7 by two partitions forming a shape of two tilted lines converging at the top.
Fig. 4 shows a tissue treatment solution immediately after performing a crushing treatment by pressing.
Fig. 5 is a schematic diagram illustrating a case where a sample and a digestive enzyme solution are put into the container for crushing a sample, and the container is sealed.
Fig. 6 shows a change in the sample before a crushing treatment (A) and after 2 minutes of the crushing treatment (B). Most of the skeletal muscle tissue is found to be suspended by the 2 minutes of crushing.
Fig. 7 illustrates comparison of the numbers of myoblast cells isolated by Comparative Example 6 (A) and Example 5 (B). The number of cells is found to be significantly increased by combining the 2 minutes of the crushing treatment with a shredding treatment (B).
Fig. 8 shows a sheet-shaped cell culture obtained by Example 6.

### Description of Embodiments

Hereinafter, the present invention will be described based on preferred embodiments of the present invention.

### [Method for isolating living cells]

The present invention includes a method for isolating living cells from a tissue derived from a living body, the method including a step of crushing the collected tissue by pressing.

The cells isolated by the method of the present invention are high in the number of collected living cells and viability, and include a large proportion of stem cells.

Hereinafter, each step of the present embodiment will be described.

### <Step of crushing by pressing>

In the present disclosure, the tissue derived from a living body is not particularly limited as long as it is derived from a living body, and examples thereof include a muscle tissue, an adipose tissue, a skin tissue, a cartilage tissue, a tendon tissue, a ligament tissue, a soft tissue, a vascular tissue, a brain tissue, a cardiovascular tissue, a gastrointestinal tissue, a metabolic system tissue, a lymphoid tissue, a bone marrow tissue, and blood. The tissue derived from a living body is preferably a muscle tissue, an adipose tissue, a bone marrow tissue, or blood, and more preferably a skeletal muscle tissue.

The living cells in the present disclosure can include any living cells isolated from the tissue derived from a living body. Examples of the living cells include, but are not limited to, cardiomyocytes, fibroblast cells, epidermal cells, endothelial cells, liver cells, pancreatic cells, kidney cells, adrenal cells, periodontal ligament cells, gingival cells, periosteal cells, skin cells, synovial cells, cartilage cells, and the like, and stem cells (for example, myoblast cells (for example, skeletal myoblast cells), muscle satellite cells, mesenchymal stem cells (for example, mesenchymal stem cells derived from bone marrow, an adipose tissue, peripheral blood, skin, hair roots, a muscle tissue, an endometrial membrane, placenta, or umbilical cord blood, and the like), tissue stem cells such as cardiac stem cells, embryonic stem cells, and the like). The living cells in the present invention may be localized between a plurality of membranes which are in contact with each other in a plane by being in direct contact with or adjacent to the plurality of membranes, thereby being surrounded by the membranes. Preferable examples of the living cells in the present invention include CD56-positive cells in a skeletal muscle tissue such as myoblast cells and muscle satellite cells or mesenchymal stem cells derived from bone marrow, an adipose tissue, or peripheral blood.

The tissue derived from a living body used in the present invention can be derived from any organisms. The organisms are not particularly limited, and examples thereof include a human, a non-human primate, a rodent (a mouse, a rat, a hamster, a guinea pig, or the like), a dog, a cat, a pig, a horse, a cow, a goat, and a sheep. In a case where cells isolated from the tissue derived from a living body used in the present invention are used for transplantation, a rejection response can be prevented by using autologous cells isolated using a tissue derived from a living body that is collected from a subject of the transplantation (recipient). However, it is also possible to use cells derived from a different species or non-self cells derived from the same species that are isolated using a tissue derived from a living body of a different species or a non-self tissue derived from a living body of the same species.

In the pressing in the present invention, the tissue derived from a living body is pressed from the outside, loosening binding in the tissue derived from a living body such as binding between tissues, between the tissue and cells, and/or between the cells, and the pressing is continued to crush the tissue. As a result, the living cells constituting the tissue derived from a living body, particularly, cells that are localized inside the tissue, can be isolated without being damaged.

Therefore, in a case where the pressing of the collected tissue derived from a living body in the present invention can loosen the binding in the tissue, the pressing may be directly applied to the tissue derived from a living body or may be applied through a container into which the tissue derived from a living body is put from the outside of the container.

In the case of directly pressing the tissue derived from a living body, the pressing can be performed in a state in which the tissue derived from a living body is put into, for example, a petri dish containing a physiologically acceptable liquid or the like, by fixing the tissue with forceps and pressing the tissue multiple times. In the case of pressing through a container, the pressing can be performed by putting the tissue derived from a living body into a container (for example, a bag-shaped container) together with a physiologically acceptable liquid, fixing the container, and pressing the container itself multiple times.

The pressing in the present invention is preferably performed through the container containing the tissue derived from a living body. The pressing through the container allows pressurization by a water flow generated by movement of the physiologically acceptable liquid in the container in addition to the direct pressurization of the tissue derived from a living body in the container, whereby the tissue derived from a living body can be evenly and efficiently crushed.

As a method of physically isolating cells from the tissue derived from a living body, for example, a grinding treatment or an ultrasonic treatment is generally used. However, these treatments directly disrupt the structure inside the tissue and homogenize the tissue to isolate the cells, and thus are different from the crushing of the present invention performed by the pressing.

Any method can be used for the pressing as long as the method causes loosening of the binding in the tissue and generates a shearing force to the extent that does not destruct the living cells. The method may be performed by hand or mechanically, and the mechanical method is preferable in order to perform the crushing without raising the temperature. In an aspect of the present invention, in the case of pressing through a container, a paddle-type homogenizer is typically preferable.

In an aspect of the present invention, in the case of pressing through a container, the same site of the container may be continuously pressed, however, it is preferable that at least two or more sites of the container are sequentially pressed, for example, two sites are alternately pressed, since the tissue derived from a living body in the container can be evenly pressurized, and the water flow generation in the container can be facilitated. Since the water flow generated in this manner not only pressurizes the tissue derived from a living body but also agitates the tissue, even and efficient crushing is possible.

In an aspect of the present invention, a step of cutting the collected tissue derived from a living body, for example, a step of cutting the tissue into a size of about 1 to 10 mm², preferably about 5 mm², can be optionally provided before the step of crushing by pressing.

As the paddle-type homogenizer in the present invention, a device that crushes a sample in a container by pressing the container containing the sample between paddles reciprocally moving towards the container containing the sample and a pressing part provided to face the paddles can be used. For example, a homogenizer that includes a sample accommodating part in which a container containing a sample can be placed between an openable door and a paddle surface and alternately pressed between the two paddles provided in the paddle surface in the sample accommodating part and the openable door (pressing part) can be used as the device. Examples of such device include homogenizers such as Pro media SH-IIM (ELMEX LIMITED, code No. SH-2M), a bag homogenizer BH-W (AS ONE Corporation), and BagMixerR (InterScience, product code 021-110). As a paddle of such homogenizer, a flat-plate paddle or an uneven paddle can be used, and it is preferable that the pressing is alternately performed by the flat-plate paddle and the uneven paddle for sufficient crushing of the tissue derived from a living body. Examples of the homogenizer include the homogenizer described in JP H07-284679 A.

The crushing in the present invention may be performed to the extent that a part of the tissue derived from a living body is dispersed or suspended in the physiologically acceptable liquid, and the crushing is not required to be performed to the extent that the tissue derived from a living body is homogeneously dispersed or suspended. The crushing may be performed so that, for example, an average tissue length of the tissue derived from a living body is about 4 mm² or less, about 3 mm² or less, about 2 mm² or less, or about 1 mm² or less. The crushing is, for example, a treatment of the tissue using the paddle-type homogenizer for 30 seconds to 30 minutes, 2 to 15 minutes, or 3 to 15 minutes, and the treatment is preferably performed for 1 to 5 minutes from the viewpoint of obtaining a constant amount of collected living cells while preventing a decrease in viability.

The physiologically acceptable liquid is not particularly limited as long as the cells contained in the tissue derived from a living body can survive therein, and examples thereof include a proteolytic enzyme solution, water, physiological saline, various buffer solutions (for example, PBS, HBSS, and the like), and various liquid media (for example, DMEM, MEM, F12, DME, RPMI1640, MCDB (MCDB102, 104, 107, 131, 153, 199, and the like), L15, SkBM, RITC80-7, DMEM/F12, and the like). The physiologically acceptable liquid may contain an antimicrobial agent such as an antibiotic substance and an antifungal agent for suppression of the growth of microorganisms incorporated therein.

The proteolytic enzyme solution may contain a proteolytic enzyme such as collagenase or matrix metalloproteinase that breaks down a fibrous tissue, and trypsin or TrypLE Select (Life Technologies Corporation) that dissociates adhesion between cells or adhesion between cells and a culture substrate. The proteolytic enzyme solution may contain one or two or more kinds of the proteolytic enzyme. For example, the proteolytic enzyme solution may contain both collagenase and trypsin. A concentration of collagenase may be 0.01 to 0.25% (W/V), and a concentration of trypsin may be 0.001 to 0.25% (V/V). Specifically, a trypsin-EDTA (1×) solution (Life Technologies Corporation) can be used as trypsin, and collagenase A (Roche Applied Science), Collagenase Lyophilized (derived from Clostridium Histolyticum, Life Technologies Corporation), or Liberase MNP-S (Roche Applied Science) can be used as collagenase. It is preferable that the proteolytic enzyme is used in an excess amount with respect to the amount of proteins contained in the tissue derived from a living body. For example, in a case where a weight of a skeletal muscle tissue is 1 to 2 g, an excess amount of the proteolytic enzyme is added when 20 mL of TrypLE Select containing collagenase A at a concentration of 0.5 mg/L is added thereto.

The proteolytic enzyme solution may contain a calcium ion chelator EDTA or EGTA. A concentration of EDTA or EGTA may be 0.02 to 0.1% (W/V). In a case where the proteolytic enzyme does not contain EDTA or EGTA, it is preferable that a solution of EDTA or EGTA is prepared separately from the proteolytic enzyme, and the tissue derived from a living body is, for example, soaked in the solution of EDTA or EGTA before being added to the proteolytic enzyme solution. The concentration of EDTA or EGTA may also be 0.02 to 0.1% (W/V) in this case.

In an aspect of the present invention, in the case of pressing through the container, a volume of the physiologically acceptable liquid to be put into the container is not particularly limited, as long as the tissue derived from a living body can be evenly pressed and crushed. In a case of using a commercially available homogenization bag such as a PYXON-20 series (ELMEX LIMITED, code No. PX0020 or the like), the volume of the physiologically acceptable liquid can be, for example, 300 mL or less, 200 mL or less, 100 mL or less, 75 mL or less, 50 mL or less, 25 mL or less, 20 mL or less, 10 mL or less, or 7.5 mL or less, and the volume is preferably 15 mL from the viewpoint that a skeletal muscle tissue can be evenly crushed and dispersed. The cut skeletal muscle tissue and the physiologically acceptable liquid may be separately put into the container or may be mixed in another container in advance and then put into the container.

A temperature of the liquid is not particularly limited as long as the viability of the myoblast cells and the number of the collected living cells are not decreased. For example, the temperature can be 4 to 37°C, 10 to 30°C, or 15 to 25°C, and typically, the temperature is room temperature.

In an aspect of the present invention, in the case of pressing through the container, the container used in the step of crushing (may also be referred to as a container for crushing a sample) may be any container that is flexible enough to allow the sample to be crushed by the pressing from the outside of the container and is airtight and strong enough to prevent leaking of the sample containing the skeletal muscle tissue and the liquid while the container is pressed. The container can be bottle-shaped, cylindrical, tube-shaped, or box-shaped, however, the shape of the container is not limited thereto. A bag-shaped container is preferable, since the container can be evenly squeezed by the pressing, and a strength that can even withstand continuous pressing can be imparted. In a case of the bag-shaped container, the container may have a polygonal (quadrangular) shape or a shape having a curved contour with reduced corners. Specific examples of the bag-shaped container include a bottom seal bag, a side seal bag, a three side seal bag, a pillow bag, a gusset bag, a standing pouch, and a round seal bag.

In an aspect of the present invention, in the case of pressing through the container, a material having sufficient airtightness and strength to withstand continuous pressing may be used as a material for the container for crushing a sample, and a resin film or sheet is preferable. As a material for the film or sheet, for example, one or two or more selected from polystyrene, polyethylene, polypropylene, nylon, polyester, polycarbonate, and synthetic rubber can be used. The resin film or sheet may have a single-layer structure or a laminated structure in which films or sheets of the same or different materials are multi-layered. A thickness of the container is not particularly limited. In general, a container having a thickness within a range of, for example, 100 µm or less is used.

A dimension of the container for crushing a sample can be appropriately selected by those skilled in the art according to the pressing method or the device that is used, and the dimension is not particularly limited. For example, in a case where the paddle-type homogenizer is used, a commercially available homogenization bag can be used, in addition to a quadrangular bag-shaped container created by heat sealing both side edges and the bottom edge of a polyethylene sheet. As the commercially available homogenization bag, for example, a PYXON-20 series (ELMEX LIMITED, code No. PX0020 or the like), a SANISPECK test bag (AS ONE Corporation, catalog No. 2-6391-02), RollBagR (InterScience, Ref. 145 040), BagFilter (InterScience, Ref. 111 720), or the like can be used. The commercially available homogenization bag may or may not include a filter in the bag, and a homogenization bag including a filter is considered to exhibit a greater shearing force due to friction that can be generated between the skeletal muscle tissue and the filter in the bag.

In an aspect of the present invention, the pressing may be applied to the container containing the sample through a buffer material. That is, the container containing the sample may be pressurized by the pressing applied to the buffer material. When the container containing the sample is pressurized by the pressing applied to the buffer material, the number of collected living cells and viability can be increased in the stem cells. It is assumed that the container can be pressurized by mitigating the impact during the pressing by the buffer material, whereby the binding in the tissue can be loosened without destructing the cells. The buffer material is not particularly limited as long as it is a material capable of absorbing the impact during the pressing. For example, an elastic body such as rubber, fabric, and Styrofoam, or a bag-shaped container containing a liquid, a gel, or a powder can be used. The bag-shaped container containing a liquid is preferable as the buffer material, from the viewpoint of the ability to relay the pressure applied by the pressing to the container containing the sample by tightly adhering to the container.

In an aspect of the present invention, in the case of pressing through the container, a dimension of the buffer material can be appropriately selected, as long as it is a dimension that allows the impact during the pressing to be mitigated and the buffer material to tightly adhere to the container containing the sample. For example, in a case where the paddle-type homogenizer is used, it is preferable that the bag-shaped container containing a liquid has the same dimension as that of the container for pressing the sample. A volume of the liquid contained in the bag-shaped container is not particularly limited, as long as the pressurization can be performed by mitigating the impact during the pressing. Those skilled in the art can appropriately select the volume according to the pressing method or the device that is used, and in a case where a commercially available homogenization bag such as a PYXON-20 series (ELMEX LIMITED, code No. PX0020 or the like) is used to perform the pressing, the volume may be about 20 mL to 300 mL, 50 mL to 200 mL, or 75 mL to 100 mL, and a volume of 100 mL is preferable.

The tissue derived from a living body that has undergone the step of crushing includes a larger number of collected living cells and higher viability and contains a larger proportion of stem cells than in a case of performing isolation without the step of crushing. After undergoing an enzyme treatment (an enzyme digestion) step described later, the number of collected living cells and viability can be 1.0 × 10³ cells or more, 1.0 × 10⁴ cells or more, 1.0 × 10⁵ cells or more, or 1.0 × 10⁶ cells or more, and 80% or more, 85% or more, 90% or more, or 95% or more, respectively. In one aspect, the proportion of the stem cells after three passages of a cell population that has undergone the enzyme treatment step described later can be 60% or more, 65% or more, 70% or more, 75% or more, 80% or more, 85% or more, 90% or more, or 95% or more.

The number of collected living cells and viability can be determined using any known method. Examples of the method include counting the total number of collected cells and the number of living cells using a method of double staining the cells or the like and dividing the number of living cells by the total number of cells. A proportion of the myoblast cells can be determined using any known method. Examples of the method include labeling the myoblast cells and/or muscle satellite cells with a specific antibody and dividing the number of positive cells to which the antibody is bound by the counted total number of cells. The counting of the cells can be performed by microscopic observation of a specimen (a sample) stained with a specific antibody, image analysis of a microscopic image, flow cytometry analysis of a cell population stained with a specific antibody, or the like. For example, in a case where the stem cells are myoblast cells, examples of a marker specific to the cells include, but are not limited to, CD56, α7 integrin, myosin heavy chain IIa, myosin heavy chain IIb, myosin heavy chain IId (IIx), MyoD, Myf5, and myogenin. In a case where the stem cells are muscle satellite cells, examples of a marker specific to the cells include, but are not limited to, CD56, CD34, Myogenin, Myf5, and Pax7.

### <Step of shredding (mincing)>

The isolation method of the present invention may or may not include a step of shredding after the step of crushing. In a case where the step of shredding is not included after the step of crushing, the enzyme treatment step described later may be performed after the step of crushing. The shredding in the present disclosure refers to obtaining a smaller size of tissue fragments from the tissue of the subject using physical means such as an instrument. In a case where the step of shredding is performed after the step of crushing, a solution of the tissue crushed by the pressing is transferred to a container for performing the shredding such as a petri dish, and the crushed skeletal muscle tissue is further shredded. Before starting the shredding, connective tissues (white tissues) in the solution of the crushed tissue may be removed.

Instead of the step of shredding, the isolation method in an aspect of the present invention may be combined with a known physical cell isolation method after the step of crushing, such as a step of grinding or a step of ultrasonic crushing. In the present invention, the binding between tissues can be loosened by performing the step of crushing before the step of grinding or the step of ultrasonic crushing, and therefore, the isolation can be performed under a milder condition than that for general grinding or ultrasonic crushing. As a result, a larger proportion of stem cells can be obtained with a larger number of collected living cells and higher viability than in a case of performing isolation without the step of crushing.

### <Enzyme treatment step>

An enzyme treatment (an enzyme digestion) step may be performed after the step of shredding. In the enzyme treatment step, a treatment product of the tissue derived from a living body is subjected to an enzyme treatment, and cells are collected from an enzyme treatment solution. The enzyme treatment can be performed by soaking the treatment product of the tissue derived from a living body in a proteolytic enzyme solution for a predetermined amount of time. The proteolytic enzyme solution is as described above.

A treatment temperature for the enzyme treatment depends on the optimum temperature, the deactivation temperature, and the like of the enzyme that is used, however, the treatment temperature is preferably 35 to 40°C in general. It is preferable that a volume of the enzyme solution is a volume in which the entire treatment product of the tissue derived from a living body is soaked in order to disrupt the connective tissues in the entire treatment product. It is also preferable that stirring is performed during the enzyme treatment.

A method for collecting cells from the enzyme treatment solution is not particularly limited, and examples thereof include a method of collecting a precipitate obtained by allowing the enzyme treatment solution to stand or by centrifuging the enzyme treatment solution and a method of separately collecting a supernatant and a precipitate obtained by allowing the enzyme treatment solution to stand or by centrifuging the enzyme treatment solution, the precipitate being collected as it is and the supernatant being further subjected to filtering, and collecting a precipitate obtained by centrifuging the filtered supernatant. Furthermore, an undigested muscle tissue may be separated using a cell strainer or the like and subjected to further enzyme treatment.

The cells collected by the isolation method of the present invention may be further subjected to a culture step and a passage (a subculture) step of passaging the cultured cells. The culturing and passaging of the cells can be performed using any known method. The cells collected by the isolation method of the present invention may be further subjected to a gene introduction step of introducing a gene. The gene to be introduced is not particularly limited as long as the gene is useful for treatment of a patient to be treated. For example, the gene may be a gene for cytokine such as HGF. The introduction of the gene can be performed using any known method such as a calcium phosphate method, a lipofection method, an ultrasound introduction method, an electroporation method, a particle gun method, a method of using a virus vector such as an adenoviral vector and a retroviral vector, and a microinjection method.

### [Container for crushing sample]

Next, the container for crushing a sample in an aspect of the present invention which can be used in the case of pressing through the container will be described.

The present invention is a container for crushing a sample by pressing, the container including a container main body having an opening part and a partition which separates an upper space and a lower space of the container main body, in which at least a part of the partition has a portion which is not horizontal with respect to the bottom of the container.

In general, when the container for crushing a sample is pressed in a state of being fixed, the sample containing the tissue derived from a living body and a physiologically acceptable liquid in the container moves to a portion other than the pressed portion in the container, particularly, an upper portion of the container for crushing a sample. For example, when the container is pressed with one paddle of the paddle-type homogenizer, the sample in the container moves towards the side of the other paddle which is not in motion, and when the other paddle presses the container, the sample moves towards the side of the one paddle which is not in motion. At this time, the partition separating the upper space and the lower space in the container main body can restrict the movement of the sample to the lower space during the pressing, and as a result, the tissue derived from a living body can be effectively and directly pressurized. In addition, at least a part of the partition has a portion which is not horizontal with respect to the bottom of the container, for example, a slope (an inclined portion, a tilted portion), which causes the sample which was moving upward to move along the slope. Therefore, the partition can act as a guide for inducing the movement of the sample in the lower space to a specific direction, thereby facilitating the generation of the water flow during the pressing. Since the water flow can pressurize and agitate the tissue derived from a living body, the tissue derived from a living body can be more evenly and efficiently crushed.

A shape of the partition is not limited as long as at least a part of the shape has a portion which is not horizontal with respect to the bottom of the container. The shape may be linear or curved, and examples thereof include a shape that resembles an inverted V, a shape of two tilted lines converging at the top (, which is protruding towards the top), and a nearly arc shape, and in a case where the partition is in a planar shape, examples of the shape include a tapered structure such as a nearly trigonal pyramid shape or a nearly dome shape. The partition may be formed of any number of partitions, for example, one or a plurality of (2, 3, 4, 5, or 6) partitions.

A volume ratio between the upper space and the lower space formed by the partition is not particularly limited as long as the movement of the tissue derived from a living body is restricted compared to the movement before the partition is provided. For example, the upper space and the lower space are separated in a volume ratio of 1 to 100:1, 1 to 50:1, 1 to 25:1, or 1 to 10:1. The partition may be provided by any method, as long as the partition separates the upper space and the lower space. For example, the container main body may be fractionated (separated) by heat sealing, pressure bonding, or use of an adhesive, or a partition member formed of the same material as the container or any material different from that of the container may be provided to the container main body by heat sealing, pressure bonding, or adhesion.

A portion of the upper space can be gripped when, for example, an openable door of a pressing-type homogenizer is closed, whereby the container for crushing a sample can be fixed.

In a preferred aspect of the present invention, the container has a communicating hole through which the upper space and the lower space can communicate with each other. The sample can be put into and/or taken out of the lower space through the communicating hole. The communicating hole may be provided at any position as long as the sample can be put into the lower space through the upper space, and the sample can be easily taken out when the communicating hole is provided at the apex of a protruding part of the lower space. In particular, in a case where the sample is sucked out (aspirated) by inserting the tip of a pipette to the communicating hole when taking out the sample, the sample in the lower space can be taken out without residual sample in the lower space. However, the communicating hole is not necessarily required to realize both the putting in and taking out of the sample, and even when a communicating hole 5 which is provided for the purpose of putting in the sample is at an arbitrary position other than the protruding part, the apex of the protruding part may be cut after the crushing treatment, and the sample may be taken out by inserting the tip of the pipette therein.

In a preferred aspect of the present invention, in a case where the communicating hole is provided, the container has a sealed part that prevents the movement of the sample to the upper space. The sealed part may be any sealing means as long as it causes the sample to be contained in the lower space without moving to the upper space through the communicating hole during the pressing, and the sealed part may be provided by heat sealing, pressure bonding, or attaching a sealing member such as a zipper, which is formed of the same material as the container or any material different from that of the container, to the container main body, or the container main body can be sealed, for example, by heat sealing, pressure bonding, or use of an adhesive.

Hereinafter, the container for crushing a sample that can be suitably used in the step of crushing in the present invention will be described based on the drawings. The container according to the present invention will be described in detail with reference to the drawings. Note that the present invention is not limited to the following embodiments and can be variously modified and implemented within the scope of the gist of the present invention.

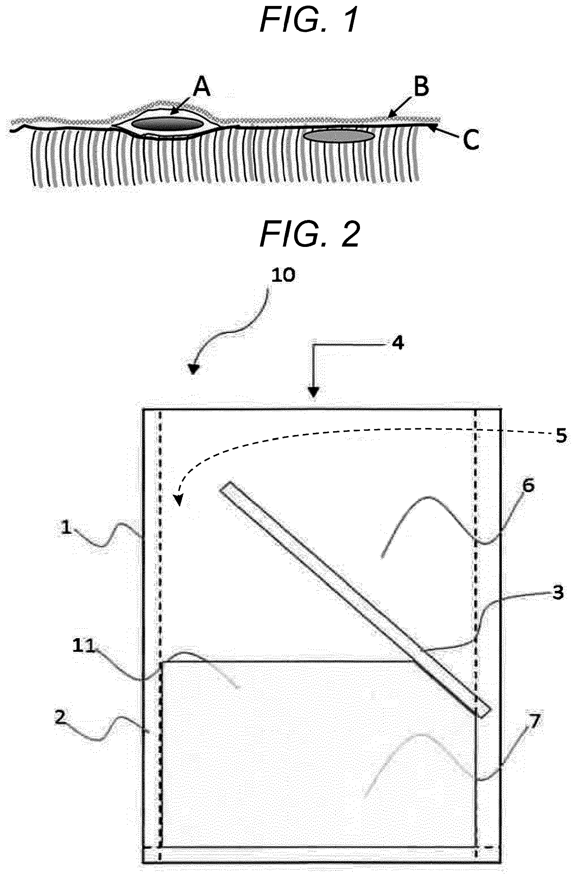

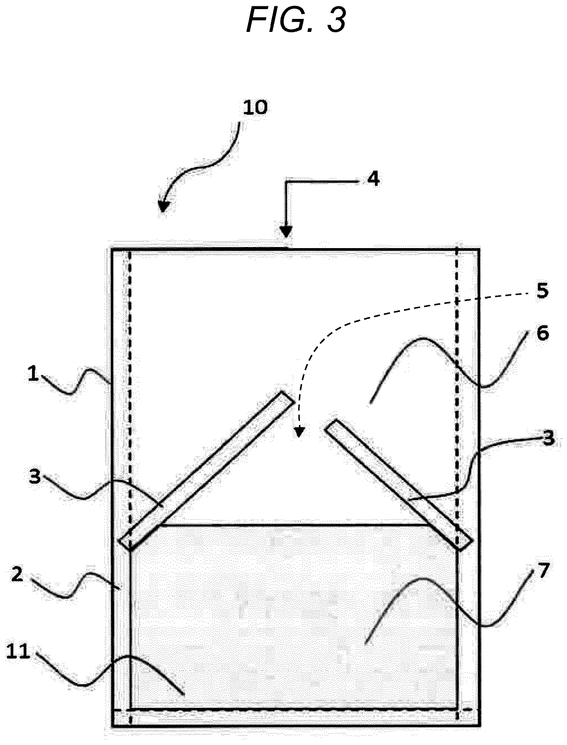

As shown in Figs. 2 and 3, in an aspect of the present invention, a container 10 includes a container main body 1 having an opening part 4 and a partition 3 which separates the container main body 1 into an upper space and a lower space, the container having one partition (Fig. 2) or two partitions (Fig. 3) that are not horizontal with respect to the bottom of the container. By providing such partition that is not horizontal with respect to the bottom of the container, a protruding part which protrudes towards the opening part is formed on the left side of a lower space 7 in Fig. 2 and in the middle of the lower space 7 in Fig. 3.

The partition 3 does not completely separate an upper space 6 and the lower space 7, and the partition may have a communicating hole 5 through which the upper space 6 and the lower space 7 can communicate with each other. A sample containing a tissue derived from a living body and a physiologically acceptable liquid can be put into and/or taken out of the lower space 7 through the communicating hole 5. After putting in the sample, the communicating hole 5 is sealed by heat sealing or the like. When a part of the upper space 6 is fixed in this state, and the lower space 7 is pressed, the movement of the sample solution to the upper space 6 is restricted by the partition 3, and the sample with the restricted movement moves along the slope of the partition 3, whereby the generation of the water flow can be facilitated in the lower space 7 during the pressing. After the crushing, the container main body is horizontally cut so that the apex of the protruding part of the container main body is opened, and the sample can be sucked out by inserting the tip of a pipette to the lower space 7 through an opening thus formed.

Fig. 2 is a container preferably used when the same site in the lower space 7 is pressed, and the partition 3 is provided in a manner that the protruding part is formed in the upper left portion of the lower space 7.

Fig. 3 is a container preferably used when the left side and the right side of the lower space 7 are alternately pressed at a constant interval, and the two partitions 3 are arranged in a manner that the protruding part having a shape of two tilted lines converging at the top is formed with the communicating hole 5 in the middle of the lower space 7.

### [Method for producing graft]

Next, a method of the present invention for producing a graft will be described.

In the present invention, a "graft" refers to a construct to be transplanted into a living body, particularly, a construct for transplantation containing cells as a constituent. In a preferred aspect, the graft is a construct for transplantation not containing a structure other than cells and a substance derived from the cells (for example, a scaffold). Examples of the graft in the present disclosure include, but are not limited to, a sheet-shaped cell culture, a spheroid, and a cell aggregate. A sheet-shaped cell culture or a spheroid is preferable, and a sheet-shaped cell culture is more preferable.

In the present disclosure, the "sheet-shaped cell culture" refers to cells connected to each other forming a sheet shape. The cells may be connected to each other directly (including connection through a cellular element such as an adhesion molecule) and/or through a mediator. The mediator is not particularly limited as long as it is a substance that can at least physically (mechanically) connect the cells to each other, and examples thereof include extracellular matrix. It is preferable that the mediator is derived from cells, particularly, from cells constituting the sheet-shaped cell culture. Although the cells are at least physically (mechanically) connected, the cells may be further connected functionally, for example, chemically or electrically. The sheet-shaped cell culture may be formed of one cell layer (monolayer) or two or more cell layers (layered construct (multilayer), for example, double-layered, triple-layered, quadruple-layered, quintuple-layered, or sextuple-layered). Furthermore, the cells in the sheet-shaped cell culture do not show a clear layered structure, and the sheet-shaped cell culture may have a three-dimensional structure having a thickness exceeding the thickness of one cell. For example, in a vertical cross section of the sheet-shaped cell culture, the cells may exist in a non-uniformly arranged state (for example, as a mosaic), instead of being uniformly aligned in the horizontal direction.

It is preferable that the graft of the present disclosure, especially the sheet-shaped cell culture, does not contain a scaffold (support). The scaffold is used in this technical field to maintain physical cohesiveness of a graft such as the sheet-shaped cell culture by attaching the cells on the surface and/or inside thereof, and a polyvinylidene difluoride (PVDF) membrane or the like is known as an example. However, the graft of the present disclosure can maintain the physical cohesiveness thereof without the presence of the scaffold. In addition, it is preferable that the graft of the present disclosure is only formed of substances derived from the cells constituting the graft and does not contain other substances.

The cells may be cells derived from a different species or cells derived from the same species. Here, when the graft is used for transplantation, the "cells derived from a different species" refer to cells derived from an organism of a species different from that of the recipient of the transplantation. For example, in a case where the recipient is a human, cells derived from a monkey or a pig are the cells derived from a different species. Furthermore, the "cells derived from the same species" refer to cells derived from an organism of the same species as the recipient. For example, in a case where the recipient is a human, human cells are the cells derived from the same species. The cells derived from the same species include self-derived cells (also referred to as self cells or autologous cells), that is, cells derived from the recipient, and non-self cells derived from the same species (also referred to as allogeneic cells). The self-derived cells are preferable in the present disclosure, since a rejection response does not occur when they are transplanted. However, it is also possible to use the cells derived from a different species or the non-self cells derived from the same species. When the cells derived from a different species or the non-self cells derived from the same species are used, immunosuppression treatment may be needed in order to suppress a rejection response. In the present specification, cells other than the self-derived cells, that is, the cells derived from a different species and the non-self cells derived from the same species may be collectively referred to as non-self-derived cells. In an aspect of the present disclosure, the cells are autologous cells or allogeneic cells.

The culture substrate is not particularly limited as long as the cells can form a cell culture thereon, and examples thereof include containers of various materials and/or shapes and a solid or semisolid surface in a container. It is preferable that the structure and the material of the container do not allow permeation of a liquid such as a culture solution. Examples of the material include, but are not limited to, polyethylene, polypropylene, Teflon (registered trademark), polyethylene terephthalate, polymethylmethacrylate, Nylon 6,6, polyvinyl alcohol, cellulose, silicon, polystyrene, glass, polyacrylamide, polydimethylacrylamide, and metal (for example, iron, stainless steel, aluminum, copper, and brass). It is also preferable that the container preferably has at least one flat surface. Examples of the container include, but are not limited to, a culture container having a bottom formed of a culture substrate on which the cell culture can be formed and a liquid impermeable side surface. Specific examples of the culture container include, but are not limited to, a cell culture dish and a cell culture bottle. The bottom of the container may be transparent or opaque. When the bottom of the container is transparent, the cells can be observed and counted from the bottom of the container. In addition, the container may have a solid or semisolid surface therein. Examples of the solid surface include plates or containers of the various materials described above, and examples of the semisolid surface include a gel and a soft polymer matrix. The culture substrate may be prepared using the above materials, or a commercially available culture substrate may be used.

Preferred examples of the culture substrate include, but are not limited to, a substrate having an adhesive surface which is suitable for formation of the sheet-shaped cell culture and a substrate having a surface with low adhesiveness and/or a substrate having a uniform well structure which is suitable for formation of spheroids. Specific examples of the substrate in a case of forming the sheet-shaped cell culture include a substrate having a surface coated with a hydrophilic compound such as polystyrene subjected to corona discharge treatment, collagen gel, and a hydrophilic polymer, and a substrate having a surface coated with an extracellular matrix such as collagen, fibronectin, laminin, vitronectin, proteoglycan, and glycosaminoglycan or with a cell adhesion factor such as the cadherin family, the selectin family, and the integrin family. Such substrates are also commercially available (for example, Corning(R) TC-Treated Culture Dish, Corning Incorporated). Specific examples of the substrate in a case of forming the spheroids include a substrate having a surface coated with a non-cell-adhesive compound, for example, a hydrogel such as soft agar, temperature-responsive gel obtained by crosslinking poly(N-isopropylacrylamide) (PIPAAm) with polyethylene glycol (PEG) (commercially available under the name Mebiol Gel), polyhydroxyethyl methacrylate (polyHEMA), and a 2-methacryloyloxyethyl phosphorylcholine (MPC) polymer, and/or a substrate having a surface with a uniform concave and convex structure. Such substrates are also commercially available (for example, EZSPHERE(R)). The culture substrate may be entirely or partially transparent or opaque.

The surface of the culture substrate may be coated with a material of which a physical property changes in response to a stimulus such as temperature and light. The material is not limited, and a known material such as a temperature-responsive material formed of a homopolymer or a copolymer such as a (meth)acrylamide compound, an N-alkyl substituted (meth)acrylamide derivative (for example, N-ethylacrylamide, N-n-propylacrylamide, N-n-propylmethacrylamide, N-isopropylacrylamide, N-isopropylmethacrylamide, N-cyclopropylacrylamide, N-cyclopropylmethacrylamide, N-ethoxyethylacrylamide, N-ethoxyethylmethacrylamide, N-tetrahydrofurfurylacrylamide, N-tetrahydrofurfurylmethacrylamide, or the like), an N,N-dialkyl substituted (meth)acrylamide derivative (for example, N,N-dimethyl (meth)acrylamide, N,N-ethylmethylacrylamide, N,N-diethylacrylamide, or the like), a (meth)acrylamide derivative having a cyclic group (for example, 1-(1-oxo-2-propenyl)-pyrrolidine, 1-(1-oxo-2-propenyl)-piperidine, 4-(1-oxo-2-propenyl)-morpholine, 1-(1-oxo-2-methyl-2-propenyl)-pyrrolidine, 1-(1-oxo-2-methyl-2-propenyl)-piperidine, 4-(1-oxo-2-methyl-2-propenyl)-morpholine, or the like), and a vinyl ether derivative (for example, methyl vinyl ether), and a light-responsive material such as a light-absorbing polymer having an azobenzene group, a copolymer of a vinyl derivative of triphenylmethane leucohydroxide and an acrylamide-based monomer, and an N-isopropylacrylamide gel containing spirobenzopyran can be used (for example, see JP H2-211865 A and JP 2003-33177 A). A physical property of these materials such as hydrophilicity and hydrophobicity is changed upon receiving a certain stimulus, and detachment of the cell culture attached to the materials can be promoted. Culture dishes coated with the temperature-responsive material are commercially available (for example, UpCell(R) of CellSeed Inc.), and these can be used in the production method of the present disclosure.

The culture substrate may be in various forms. Furthermore, an area of the culture substrate is not particularly limited, and the area may be, for example, about 1 cm² to about 200 cm², about 2 cm² to about 100 cm², or about 3 cm² to about 50 cm². An example of the culture substrate is a round culture dish having a diameter of 10 cm. In this case, the area is 56.7 cm². The culture surface may be flat or may have a concave and convex structure. In a case of having the concave and convex structure, the concave and convex structure is preferably uniform.

The culture substrate may be coated with a component derived from blood and/or a cell-adhesive component in order to form a graft, particularly, the sheet-shaped cell culture, with a higher density. "Being coated with a component derived from blood and/or a cell-adhesive component" refers to a state in which a component derived from blood such as serum and/or a cell-adhesive component adheres to the surface of the culture substrate, and the state can be obtained, for example, by treating the culture substrate with the component derived from blood and/or the cell-adhesive component, but the method is not limited thereto. The treatment with the component derived from blood and/or the cell-adhesive component includes, for example, bringing the serum and/or the cell-adhesive component into contact with the culture substrate and incubating the culture substrate for a predetermined period as necessary. The serum and/or the cell-adhesive component used for the coating may be serum of a species which is the same as the species from which the seeded cells are derived (allogeneic serum) or serum of a different species (heterologous serum), for example, FBS. The allogeneic serum is preferable, and serum obtained from the individual from which the seeded cells are derived (autologous serum) is more preferable. Other examples of the component derived from blood include albumin and a platelet lysate. Examples of the cell-adhesive component used for the coating include an extracellular matrix such as collagen, fibronectin, laminin, vitronectin, proteoglycan, and glycosaminoglycan, the cadherin family, the selectin family, and the integrin family.

Seeding of the cells into the culture substrate can be performed by any known method and condition. For example, the seeding of the cells into the culture substrate may be performed by injecting a cell suspension obtained by suspending the cells in the culture solution into the culture substrate (culture container). In the injection of the cell suspension, an instrument suitable for the operation of the cell suspension injection, such as a dropper and a pipette, can be used. The seeding of the cells is performed at a seeding density at which the sheet-shaped cell culture can be formed, and the density can be different depending on the desired cells, however, an appropriate density can be selected by those skilled in the art using a method known in the technical field.

Examples of a high density can include a density reaching confluence, in other words, a density at which the cells are expected to cover the entire adhesive surface of the culture container when seeded, for example, a density at which the cells are expected to contact each other, a density at which contact inhibition occurs, or a density at which cell proliferation substantially ceases by the contact inhibition when the cells are seeded, or higher. The upper limit of the seeding density is not particularly limited, however, when the density is excessively high, the number of dead cells increases, which is inefficient. In an aspect of the present disclosure, the seeding density can be about 5.0 × 10⁵ cells/cm² to about 1.0 × 10⁷ cells/cm², about 5.0 × 10⁵ cells/cm² to about 5.0 × 10⁶ cells/cm², about 5.0 × 10⁵ cells/cm² to about 3.0 × 10⁶ cells/cm², about 1.0 × 10⁶ cells/cm² to about 1.0 × 10⁷ cells/cm², about 1.0 × 10⁶ cells/cm² to about 5.0 × 10⁶ cells/cm², about 1.0 × 10⁶ cells/cm² to about 3.0 × 10⁶ cells/cm², about 1.5 × 10⁶ cells/cm² to about 1.0 × 10⁷ cells/cm², about 1.5 × 10⁶ cells/cm² to about 5.0 × 10⁶ cells/cm², about 1.5 × 10⁶ cells/cm² to about 3.0 × 10⁶ cells/cm², about 2.0 × 10⁶ cells/cm² to about 1.0 × 10⁷ cells/cm², about 2.0 × 10⁶ cells/cm² to about 5.0 × 10⁶ cells/cm², or about 2.0 × 10⁶ cells/cm² to about 3.0 × 10⁶ cells/cm². In a preferred aspect, the seeding density is about 7.5 × 10⁵ cells/cm² to 3.0 × 10⁶ cells/cm², and in another preferred aspect, the seeding density is about 1.76 × 10⁶ cells/cm² to about 2.33 × 10⁶ cells/cm².

A seeded cell population may contain other cells (fibroblast cells), as long as the desired cells are contained, and in a case where the desired cells are myoblast cells or muscle satellite cells, the cell population can further contain, for example, fibroblast cells or vascular endothelial cells. A cell population collected from a tissue may be directly used, or cryopreservation, preculture, removal of the fibroblast cells, or the like may be performed on the cell population before use. In a preferred aspect, the seeded cell population is obtained by seeding the cells isolated from the tissue derived from a living body on the culture substrate (preferably on a flat culture substrate) and performing adhesive culture, and then collecting the cell population. Cryopreservation and thawing may be performed before or after the adhesive culture. By performing the adhesive culture, formation of a high-quality graft can be achieved with a high probability in the subsequent graft formation.

In the adhesive culture step, culture conditions or the like may be based on conditions in a case of performing conventional adhesive culture. For example, the culture may be performed using a commercially available culture container for adhesive culture under conditions of 37°C and 5% CO₂. The seeding density of the cells may be any density as long as it is a density at which the formation of the adhesion between the cells and/or the adhesion between the cells and the culture substrate is not prevented. For example, the seeding density may be a subconfluence density or a density reaching confluence or higher. Culture time may be time sufficient to allow the formation of the adhesion between the cells and/or the adhesion between the cells and the culture substrate, and specifically, the culture time may be, for example, about 2 to 24 hours, 2 to 12 hours, 2 to 6 hours, or 2 to 4 hours.

The culture solution used in the production method of the present invention is not particularly limited as long as survival of the cells can be maintained, and a culture solution containing amino acids, vitamins, and electrolytes as main components can be typically used. The culture solution in an aspect of the present invention is based on a basal medium for cell culture. Examples of the basal medium include, but are not limited to, DMEM, MEM, F12, DMEM/F12, DME, RPMI 1640, MCDB (MCDB 102, 104, 107, 120, 131, 153, 199, or the like), L15, SkBM, and RITC80-7. The basal medium with a standard composition may be used as it is (for example, a commercially available basal medium may be used as it is), or the composition may be suitably changed according to the type of the cells or a condition of the cells. Thus, the basal medium used in the present invention is not limited to a basal medium with a known composition, and includes a basal medium in which one or two or more of the components are added, removed, increased, or decreased. In general, a graft-forming medium may contain an additive such as serum (for example, bovine serum such as fetal bovine serum, horse serum, human serum, or the like) and various growth factors (for example, FGF, EGF, VEGF, HGF, or the like), however, in a case where the sheet-shaped cell culture is produced under xeno-free conditions, it is particularly preferable that the medium does not contain heterologous serum such as bovine serum and horse serum. In the present disclosure, graft-forming culture is performed using the graft-forming medium containing the cell-adhesive component, which allows the present disclosure to have an effect of being able to form a high-quality graft even when the graft-forming medium does not contain serum. Therefore, in a preferred aspect, the graft-forming medium does not contain serum.

In another aspect, the present invention relates to a kit for producing a graft, the kit including some or all of elements used in production of the graft, the graft particularly being the sheet-shaped cell culture, particularly, production of the sheet-shaped cell culture without proliferation culture.

The kit of the present invention may include, for example, a container for isolating the tissue derived from a living body, a physiologically acceptable liquid to be used for crushing, a homogenizer for isolating myoblast cells, cells forming the graft (for example, cryopreserved cells, cells collected by the collection method of the present invention, or the like), a culture solution, a culture dish, instruments (for example, a pipette, a dropper, forceps, or the like), and instructions regarding the method for producing the sheet-shaped cell culture (for example, a medium to which information on instructions for use, the production method, or the method of the present invention for collecting cryopreserved cells is recorded, such as a flexible disk, CD, DVD, a Blu-ray disk, a memory card, and a USB memory), but the elements in the kit are not limited thereto.

### [Treatment method]

Next, the myoblast cells of the present invention will be described.

Another aspect of the present disclosure relates to myoblast cells and/or muscle satellite cells isolated by the method of the present disclosure and a method for treating a disease in a subject in need of an effective dose of a graft produced from these cells, the method including applying the effective dose of the graft to the subject. The disease targeted for the treatment is as described above.

In the present disclosure, the term "treatment" includes all types of medically accepted prophylactic and/or therapeutic intervention that aim for curing, transient remission, or prevention of the disease. For example, the term "treatment" includes medically accepted interventions with various aims including delaying or stopping progression of a disease related to tissue abnormality, regression or disappearance of a lesion, prevention of onset of the disease, or prevention of a relapse of the disease.

In the treatment method of the present disclosure, a component that increases graft survival, engraftment, and/or function, other active ingredients useful for the treatment of the target disease, or the like can be used together with the graft of the present disclosure.

The treatment method of the present disclosure may further include a step of producing the graft of the present disclosure according to the production method of the present disclosure. The treatment method of the present disclosure may further include, before the step of producing the graft, a step of collecting the tissue derived from a living body serving as a supply source of the myoblast cells and/or the muscle satellite cells for producing the graft from a subject. In an aspect, the subject from whom the tissue serving as the supply source of the cells or the myoblast cells and/or the muscle satellite cells is collected is the same individual as the subject who receives the administration of the cell culture, the composition, or the graft. In another aspect, the subject from whom the tissue serving as the supply source of the myoblast cells and/or the muscle satellite cells is collected is another individual of the same species as the subject who receives the administration of the cell culture, the composition, or the graft. In another aspect, the subject from whom the tissue serving as the supply source of the myoblast cells and/or the muscle satellite cells is collected is an individual of a species different from that of the subject who receives the administration of the graft.

The effective dose in the present disclosure is, for example, an amount (for example, the size, the weight, or the number of the sheet-shaped cell cultures) in which the onset or relapse of the disease is suppressed, a symptom of the disease is alleviated, or the progression of the disease is delayed or stopped, and is preferably an amount in which the onset and the relapse of the disease are prevented, or the disease is cured. Furthermore, the effective dose is preferably an amount in which an adverse effect exceeding a benefit of the administration is not generated. The amount can be suitably determined by, for example, performing a test with experimental animals or animal disease models such as mice, rats, dogs, and pigs, and the method of the test is well known to those skilled in the art. In addition, the size of the tissue lesion targeted for the treatment can be an important index for determining the effective dose.

Examples of an administration method include intravenous administration, intramuscular administration, intraosseous administration, intraspinal administration, and direct application to the tissue. An administration frequency is typically one administration per treatment, however, in a case where a desired effect is not obtained, the administration may be performed multiple times. When applying to the tissue, the cell culture, the composition, or the sheet-shaped cell culture of the present invention may be fixed to the target tissue by a locking means such as a suture and a staple.

The preferred embodiments of the present invention have been described so far, however, the present invention is not limited thereto. In the present invention, each configuration can be substituted for any configuration capable of exhibiting the same function, or any configuration can be added.

### Examples

### <Prewash step>

About 3 g of a tissue was collected from a skeletal muscle collected from a pig lower limb, and the tissue was washed by being soaked in a tissue transporting solution (a 1.6 mg/mL glucose injection (Terumo Corporation), 0.1 mg/mL Gentamicin (Fuji Pharma Co., Ltd.), and 2.5 µg/mL Fungizone (Life Technologies Corporation) in Hanks' Balanced Salt Solution (HBSS, Life Technologies Corporation)).

### Comparative Example 1: Step of shredding by operation of instrument by hand

### [Comparative Example 1]

### <Step of shredding>

About 2 g of a skeletal muscle was collected from an edible pig lower limb, and the muscle was washed by being soaked in a tissue transporting solution (Hanks' Balanced Salt Solution: GIBCO, 1.45 mg/mL Glucose: Otsuka Pharmaceutical Co., Ltd., 0.1 mg/mL Gentamycin: Fuji Pharma Co., Ltd., and 2.5 µg/mL Fungizone: GIBCO).

Next, the washed skeletal muscle was shredded in 10 mL of a digestive enzyme solution (a collagenase-containing solution) at room temperature. White tissues (connective tissues) were removed from the shredded skeletal muscle.

### Comparative Example 2: Step of crushing with gentleMACS

### [Comparative Example 2]

### <Step of crushing with gentleMACS>

After cutting a skeletal muscle tissue into a size of 5 mm² using a scalpel, the skeletal muscle tissue was crushed by putting the tissue into gentleMACS C Tubes (Miltenyi Biotec K.K, Order no: 130-093-237) together with 10 mL of a digestive enzyme solution and feeding the C Tubes into gentleMACS Octo Dissociator (Miltenyi Biotec K.K, Order no: 130-093-237). The crushing with the gentleMACS Octo Dissociator was performed under the following conditions 1 and 2.
- Condition 1: (1) 3 minutes, +60 rpm, (2) 9 minutes, -30 rpm, (3) 5 minutes +/-30 rpm × 6 times, and (4) 12 minutes, -30 rpm
- Condition 2: (1) 30 seconds +1000 rpm and (2)10 seconds - 1000 rpm, 20 seconds +1000 rpm
(Note that "+" indicates forward rotation, and "-" indicates reverse rotation with respect to the direction of the blade)

### <Enzyme treatment step>

A digestive enzyme solution was added to each of the treatment products of the skeletal muscle tissues obtained in Comparative Example 1 and Comparative Example 2, and an enzymatic reaction was performed at 37°C. After completion of the reaction, the enzyme digestion product was stirred in a conical tube (referred to as a conical tube 1) and then allowed to stand, and the collected supernatant was filtered through a cell strainer (BD Falcon^{™} cell strainer, 40 µm, BD Japan) which was set on a new conical tube (referred to as a conical tube 2) and collected. The cell strainer was rinsed with a primary growth medium, and the medium was collected in the conical tube. The precipitated cells obtained by performing a centrifugal treatment on the collected filtrate were suspended by adding the primary growth medium thereto, and the suspension was seeded in a culture flask (area of the bottom: 175 cm²).

### <Culture step>

The cells collected in the enzyme treatment step were transferred to a culture flask (area of the bottom: 175 cm²) and cultured under conditions of 37°C and 5% (V/V) CO₂. After the culture, the cells were collected, and the number of the cells were counted.

The results of Comparative Example 1 and Comparative Example 2 are shown in Table 1. In Table 1, "Shredding" indicates the skeletal muscle tissue subjected to the shredding treatment performed by hand using an instrument according to the procedures of Comparative Example 1, "gentleMACS1" indicates the skeletal muscle tissue subjected to the crushing treatment under the condition 1 according to the procedures of Comparative Example 2, and "gentleMACS2" indicates the skeletal muscle tissue subjected to the crushing treatment under the condition 2.

**[Table 1]**

| Sample | Shredding | gentleMACS | gentleMACS |
|---|---|---|---|
| | | Condition 1 | Condition 2 |
| Amount of skeletal muscle subjected to treatment | 3.42 g | 3.54 g | 3.50 g |
| Viability | 100% | 100% | 100% |
| Number of collected living cells | 2.88 × 10⁴ (cells) | 3.33 × 10³ (cells) | 8.88 × 10³ (cells) |

From the results, it was understood that the number of collected living cells significantly decreased when the crushing treatment was performed mechanically using a conventional cell crushing device gentleMACS.

### Comparative Example 3: Step of shredding by operation of instrument by hand

### [Comparative Example 3]

About 3 g of skeletal muscle tissues were collected from the lower limbs of two individuals of pig (sample Nos.: 633 and 805), and after the prewash step, the tissues were shredded by the same method as in Comparative Example 1 described above.

### Example 1: Step of crushing by pressing

### [Example 1]

### <Step of crushing>

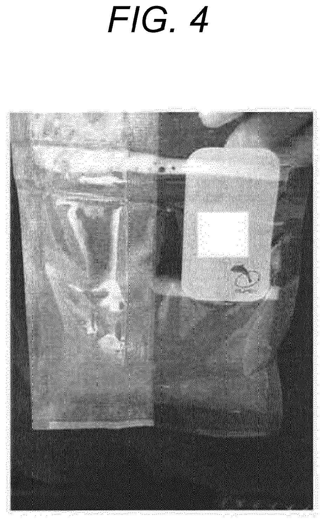

About 3 g of skeletal muscle tissues were collected from the lower limbs of the same two individuals of pig as those in Comparative Example 3 described above (sample Nos.: 633 and 805), washed by the same procedures as in the prewash step, and cut into a size of 5 mm² using a scalpel. Then, the cut tissues were put into a homogenization bag (ELMEX LIMITED, PYXON-20, code No. PX0020) together with 15 mL of a digestive enzyme solution (the digestive enzyme solution used in the step of shredding), the homogenization bag was fed to a pressing-type homogenizer (Pro media SH-IIM (ELMEX LIMITED, code No. SH-2M)), and crushing was performed for 15 minutes under the setting of the device. When a tissue that was not completely crushed was present, the tissue was transferred to a self-standing 50 mL centrifuge tube (Corning Incorporated) and allowed to stand until the skeletal muscle tissue precipitated, and the supernatant was suctioned and separated with a pipette. The tissue was then resuspended with 10 ml of the digestive enzyme solution (the digestive enzyme solution used in the step of shredding), the suspension was put into the homogenization bag again and fed to the pressing-type homogenizer, and the crushing was performed again for 15 minutes under the setting of the device. The homogenization bag containing the crushing treatment solution immediately after the crushing treatment is shown in Fig. 4.

The treatment products of the skeletal muscles obtained in Comparative Example 3 and Example 1 were subjected to a treatment according to the same procedures as in the enzyme treatment steps of Comparative Example 1 and Comparative Example 2 described above. The obtained cells were subjected to a culture step.

### <Culture step>

The cells collected in the enzyme treatment step were transferred to a culture flask (area of the bottom: 175 cm²) and cultured under conditions of 37°C and 5% (V/V) CO₂. After the culture, the cells were collected, and the number of the cells were counted. Passage culture was performed as necessary.

The results of Comparative Example 3 and Example 1 are shown in Tables 2 and 3.

In Tables 2 and 3, "Shredding" indicates the skeletal muscle tissue subjected to the shredding treatment performed by hand according to the procedures of Comparative Example 3, and "Press crushing 1" and "Press crushing 2" indicate the skeletal muscle tissues subjected to the step of crushing by pressing according to the procedures of Example 1.

**[Table 2]**

| Primary culture | | |
|---|---|---|
| Sample No. | 633 | 805 |
| Shredding | 1.5 × 10⁵ | 2.1 × 10⁴ |
| Press crushing 1 | 1.9 × 10⁴ | 7.2 × 10⁴ |
| Press crushing 2 | 1.0 × 10⁵ | - |
| Press crushing (total) | 1.2 × 10⁵ | 7.2 × 10⁴ |

**[Table 3]**

| Passage culture | | |
|---|---|---|
| Sample No. | 633 | 805 |
| Shredding | 1.4 × 10⁶ | 8.4 × 10⁵ |
| Press crushing 1 | 1.7 × 10⁶ | 2.9 × 10⁶ |
| Press crushing 2 | 3.2 × 10⁶ | - |
| Press crushing (total) | 4.9 × 10⁶ | 2.9 × 10⁶ |

It was possible to increase the number of cells in the sample of the sample No.: 633 which was crushed by pressing to be larger than that in the shredded sample by performing the passage culture. In addition, it was possible to increase the number of cells in the sample of the sample No.: 805 to be larger than that in the shredded sample both when the primary culture was performed and when the passage culture was performed. Since performing the passage culture allowed a larger number of myoblast cells to be cultured than in the shredded tissue, it is suggested that a large number of muscle satellite cells can be isolated by the treatment of crushing by pressing in addition to the myoblast cells. Furthermore, a significantly larger number of cells were obtained in the treatment of crushing by pressing than in a general cell crushing treatment (gentleMACS), and therefore, it was clear that the treatment of crushing by pressing was suitable for isolation of stem cells.

### [Comparative Example 4]

About 3 g of a skeletal muscle tissue was collected from the lower limb of an individual of pig, and after the prewash step, the tissue was shredded by the same method as in Comparative Example 1 described above.

### Example 2: Step of crushing by pressing

### [Example 2]

Four samples of about 3 g of a skeletal muscle tissue were collected from the lower limb of an individual of pig, and after the prewash step, the samples were crushed for 5, 10, 15, and 20 minutes according to the same procedures as in Example 1 described above.

### Example 3: Step of crushing using container for crushing

### [Example 3]

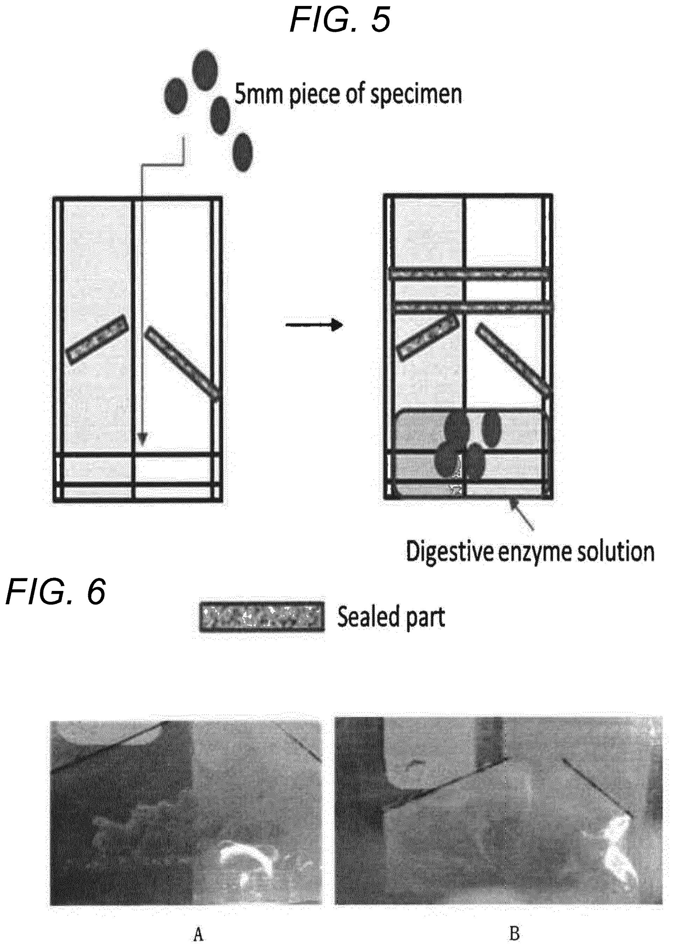

A container for isolation was produced by heat sealing a homogenization bag (ELMEX LIMITED, PYXON-20, code No. PX0020) so that a protruding part having a shape of two tilted lines converging at the top is formed in the lower space. Two samples of about 3 g of a skeletal muscle tissue were collected from the lower limb of the same individual of pig as that in Comparative Example 4 described above. As shown in Fig. 5, after performing the prewash step, the samples were cut into a size of 5 mm² using a scalpel and put into the lower space of the container for crushing together with 15 mL of the digestive enzyme solution (the digestive enzyme solution used in the step of shredding), the apex of the protruding part having the shape of two tilted lines converging at the top was sealed by heat sealing, and crushing was performed for 5 and 10 minutes using a pressing-type homogenizer (Pro media SH-IIM (ELMEX LIMITED, code No. SH-2M)).

The treatment products of the skeletal muscles obtained in Comparative Example 4 and Example 2 were subjected to a treatment according to the same procedures as in the enzyme treatment steps of Comparative Examples 1 and 2 described above. The obtained cells were subjected to a culture step.

### <Culture step>

The cells collected in the enzyme treatment step were transferred to a culture flask (area of the bottom: 175 cm²) and cultured under conditions of 37°C and 5% (V/V) CO₂. After the culture, the cells were collected, and the number of the cells were counted.

The results of Comparative Example 4 and Examples 2 and 3 are shown in Table 4.

In Table 4, "Shredding" indicates the skeletal muscle tissue subjected to the shredding treatment performed by hand according to the procedures of Comparative Example 4, each of "Press crushing_conventional container" indicates the skeletal muscle tissue subjected to the step of crushing by pressing for 5, 10, 15, or 20 minutes according to the procedures of Example 2, and each of "Press crushing_container for crushing" indicates the skeletal muscle tissue subjected to the step of crushing by pressing for 5 or 10 minutes using the container for crushing according to the procedures of Example 3.

Note that a buffer material was used in both cases of "Press crushing_conventional container" and "Press crushing_container for crushing". The buffer material was obtained by putting 100 mL of water into a homogenization bag (ELMEX LIMITED, PYXON-20, code No. PX0020) and then sealing the bag by heat sealing, and the buffer material was used by being placed on the paddle surface in the sample accommodating part of the pressing-type homogenizer (Pro media SH-IIM (ELMEX LIMITED, code No. SH-2M)).

**[Table 4]**

| Treatment method | Number of collected living cells | Viability |
|---|---|---|
| Shredding | 3.72 × 10⁴ (cells) | 100% |
| Press crushing_conventional container 5 minutes | 1.41 × 10⁴ (cells) | 100% |
| Press crushing_conventional container 10 minutes | 6.21 × 10³ (cells) | 100% |
| Press crushing_conventional container 15 minutes | 1.77 × 10³ (cells) | 100% |
| Press crushing_conventional container 20 minutes | 8.88 × 10² (cells) | 100% |
| Press crushing_container for crushing_5 minutes | 1.04 × 10⁵ (cells) | 93% |
| Press crushing_container for crushing_10 minutes | 4.70 × 10⁴ (cells) | 94% |

It was found that, in both cases of the press crushing, the number of collected cells was largest when the crushing was performed for 5 minutes, and the number of collected living cells decreased as the crushing time was increased. Although viability decreased when the container for crushing was used, since the tissues were disrupted to the deep part, sufficient amounts of the myoblast cells and/or the muscle satellite cells were isolated. The optimal isolation method was the press crushing treatment performed for 5 minutes using the container for crushing.

### [Comparative Example 5]

About 4g of a skeletal muscle tissue was collected from the lower limb of an individual of pig, and after the prewash step, the tissue was shredded by the same method as in Comparative Example 1 described above.

### Example 4: Step of crushing using container for crushing and buffer material

### [Example 4]

In the same manner as in Comparative Example 4 described above, about 4.01 g of a skeletal muscle tissue was collected from the lower limb of a pig, and after the prewash step, the tissue was cut into a size of 5 mm² using a scalpel. A container which was the same as the container for isolation produced in Example 3 was produced, 10 mL of a digestive enzyme solution was put into the lower space thereof, and the apex of the protruding part having a shape of two tilted lines converging at the top was sealed by heat sealing. Furthermore, an additional homogenization bag (ELMEX LIMITED, PYXON-20, code No. PX0020) was prepared, 100 mL of water was put thereinto, and then the bag was sealed by heat sealing, which was placed on the paddle surface in the sample accommodating part of the pressing-type homogenizer (Pro media SH-IIM (ELMEX LIMITED, code No. SH-2M)) as a buffer material. The homogenization bag containing the sample was placed on the openable door surface, and crushing was performed for 5 minutes under the setting of the device. The homogenization bag containing the sample was opened and transferred to a petri dish, and shredding was performed.

The treatment products of the skeletal muscles obtained in Comparative Example 5 and Example 4 were subjected to a treatment according to the same procedures as in the enzyme treatment steps of Comparative Examples 1 and 2 described above. The obtained cells were subjected to a culture step.

### <Culture step>

The cells collected in the enzyme treatment step were transferred to a culture flask (area of the bottom: 175 cm²) and cultured under conditions of 37°C and 5% (V/V) CO₂. After the culture, the cells were collected, and the number of the cells were counted.

### <Cell purity measurement step>

After the completion of the culture, the cells were collected, and a portion of the cells were subjected to myoblast cell purity measurement. The cells in each case were caused to react with an anti-CD56 antibody, and the proportion of CD56-positive cells (purity of myoblast cells) was measured using a flow cytometer.

The results of Comparative Example 5 and Example 4 are shown in Table 5.

In Table 5, "Shredding" indicates the skeletal muscle tissue subjected to the shredding treatment performed by hand according to the procedures of Comparative Example 4, and Example 4 indicates the skeletal muscle tissue subjected to the step of crushing by pressing using the container for crushing a sample and the buffer material and then the step of shredding by human hand, according to the procedures of Example 4.

**[Table 5]**

| Shredding method | Shredding | Example 4 |
|---|---|---|
| Amount of skeletal muscle subjected to treatment | 3.93 g | 4.01 g |
| Viability | 96% | 98% |
| Number of collected living cells | 5.24 × 10⁵ (cells) | 8.72 × 10⁵ (cells) |
| Purity (proportion of CD56-positive cells) | 96% | 98% |

It was found that, when the crushing treatment was performed by pressing using the container for crushing and the buffer material, viability, the number of collected living cells, and the proportion of the CD56-positive cells were all increased.

### [Comparative Example 6]

About 3 g of a skeletal muscle tissue was collected from the lower limb of an individual of pig, and after the prewash step, the tissue was shredded according to the same procedures as in Comparative Example 1 described above.

### Example 5: Combination of step of shredding and step of crushing

### [Example 5]

About 3 g of a skeletal muscle tissue was collected from the lower limb of the same individual of pig as that in Comparative Example 6, and crushing was performed for 2 minutes according to the same procedures as in Example 4 using the container for crushing. The homogenization bag containing the sample was opened and transferred to a petri dish, and shredding was performed.

The treatment products of the skeletal muscles obtained in Comparative Example 6 and Example 5 were subjected to a treatment according to the same procedures as in the enzyme treatment steps of Comparative Examples 1 and 2 described above. The obtained cells were subjected to a culture step.

### <Culture step>

The cells collected in the enzyme treatment step were transferred to a culture flask (area of the bottom: 175 cm²) and cultured under conditions of 37°C and 5% (V/V)

CO₂. After the culture, the cells were collected, and the number of the cells were counted.

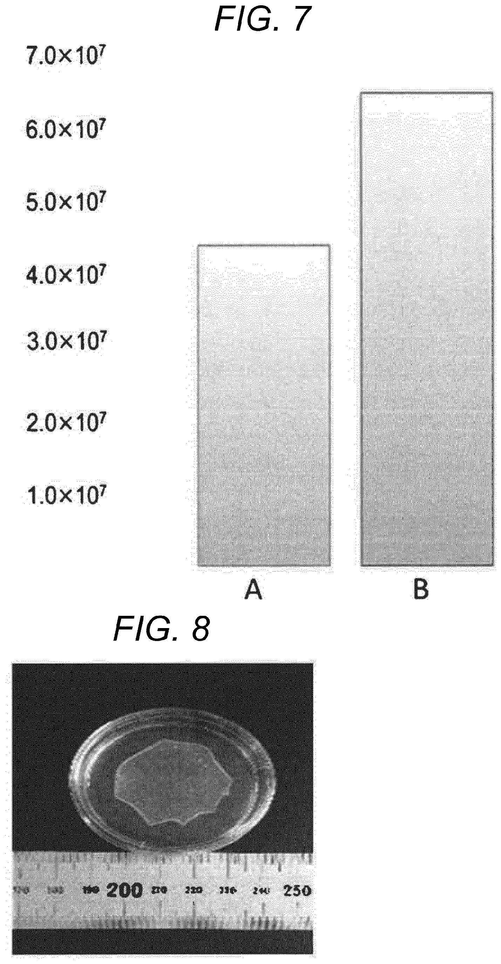

A change in the sample before the crushing and after 2 minutes of the crushing treatment are shown in Fig. 6. The results of Comparative Example 6 (A) and Example 5 (B) are shown in Fig. 7. It was clear that the number of isolated myoblast cells was significantly increased by combining the step of shredding and the step of crushing by pressing (B), compared to the case when the step of shredding was performed (A).

### Example 6: Examination of sheet-shaped cell culture obtained using isolated cells obtained in Example 5

### [Example 6]

A sheet-shaped cell culture was prepared using the myoblast cells isolated and cultured in Example 5. The myoblast cells suspended in a DMEM/F12 medium containing 20% human serum (Thermo Fisher Scientific Inc.) were seeded in a temperature-responsive culture plate (UpCell^{(R)} 12 multi-well, CellSeed Inc.) at a density of 3.7 × 10⁶ cells/well, and sheet-forming incubation was performed for 2 to 12 hours at 37°C and 5% CO₂. After the sheet-forming incubation, the medium was removed, and 700 µL of cold HBSS (+) (Thermo Fisher Scientific Inc.) was added and removed. This procedure was repeated, and after the second addition of the buffer solution, the culture was allowed to stand for 10 minutes, and then the sheet-shaped cell culture was completely detached by gentle pipetting. The sheet-shaped cell culture is shown in Fig. 8.

## Claims

1. A container for crushing a sample by pressing, the container comprising:
a container main body having an opening part; and
a partition which separates an upper space and a lower space of the container main body,
wherein at least a part of the partition has a portion which is not horizontal with respect to the bottom of the container.

2. The container according to claim 1, wherein the lower space is configured so that a protruding part which protrudes towards the opening part is formed by the partition.

3. The container according to claim 1 or 2, wherein the partition has a communicating hole at the apex thereof.

4. The container according to any one of claims 1 to 3, wherein the partition has a shape that resembles an inverted V or a shape of two tilted lines converging at the top.

5. The container according to claim 4, wherein slopes of the shape that resembles an inverted V and/or the shape of two tilted lines converging at the top are asymmetrical.

6. The container according to any one of claims 1 to 5 further comprising a sealed part.

7. The container according to any one of claims 1 to 6, wherein the partition is formed by seal formation.

8. The container according to any one of claims 1 to 7, wherein a sample is a tissue derived from a living body.

9. The container according to any one of claims 1 to 8, which is for isolating CD56-positive cells.
